# EUROPEAN PATENT SPECIFICATION

(11) **EP 4 561 983 B1**
(45) Date of publication and mention of the grant of the patent: **08.07.2026**
(21) Application number: 23745181.0
(22) Date of filing: 24.07.2023
(51) Int. Cl.: C07C 201/08, C07C 201/12, C07C 213/02, C07C 37/00

(54) **PROCESS FOR PREPARATION OF 2-CHLORO-3-FLUORO-4-ALKOXY-ANILINES AND 2-FLUORO-3-CHLOROPHENOL**
VERFAHREN ZUR HERSTELLUNG VON 2-CHLOR-3-FLUOR-4-ALKOXY-ANILINEN UND 2-FLUOR-3-CHLORPHENOL
PROCÉDÉ DE PRÉPARATION DE 2-CHLORO-3-FLUORO-4-ALCOXY-ANILINES ET DE 2-FLUORO-3-CHLOROPHÉNOL

(30) Priority: 28.07.2022 EP 22187459
(43) Date of publication of application: 04.06.2025
(73) Proprietor: Bayer Aktiengesellschaft, 51373 Leverkusen (DE)
(72) Inventor: PAZENOK, Sergii, 42799 Leichlingen (DE); VOLPIN, Giulio, 51373 Leverkusen (DE)
(74) Representative: BIP Patents
(86) International application number: PCT/EP2023/070436
(87) International publication number: WO 2024/023012

(56) References cited:
- CN-A- 112 142 567

## Description

The present invention relates to a new process for preparing 2-chloro-3-fluoro-4-alkoxy-anilines, which can be further converted to 2-fluoro-3-chlorophenol.

2-fluoro-3-chlorophenol is a valuable precursor of active fungicidal ingredients (WO2020127780). 2-chloro-3-fluoro-4-alkoxy-anilines can be used for the preparation of pharmaceutically active 1,3-benzothiazole compounds (WO2018/013774 A1).

A typical procedure for the preparation of phenol derivatives includes reaction of the corresponding commercially available aniline derivatives with nitrous acid to form a diazonium salt which then is converted *in situ* to hydroxyl group upon heating in presence of water and acid (H. E., Ungnade et al. Organic Synthesis 1943, 23, 11-13; EP1138713 A2).

The inventors of the present inventions found that 2-fluoro-3-chlorophenol can be synthesized in one step starting from 2-fluoro-3-chloroaniline following the procedure described above. However, the main drawbacks of this method are the formation of the 2-chloro-6-diazo-cyclohexa-2,4-dien-1-one as byproduct which is highly explosive and the poor yield of the outcome (20%).

CN112142567 discloses a four-step process for the preparation of 2-fluoro-3-chlorophenol starting from 1,3-dichloro-2-fluoro-4-nitrobenzene proceeding *via* a mixture of 2-chloro-3-fluoro-4-methoxy-aniline and 4-chloro-3-fluoro-2-methoxyaniline as intermediate. The first step consists in a nucleophilic substitution of one of the chlorine atoms in presence of sodium methoxide to lead to a mixture of regioisomeric compounds 3-chloro-2-fluoro-1-methoxy-4-nitrobenzene and 1-chloro-2-fluoro-3-methoxy-4-nitrobenzene. Subsequently, the nitro group of this mixture of two regioisomers is reduced to an amino group *via* catalytic hydrogenation. 2-fluoro-3-chlorophenol is then obtained by deamination of the compounds 2-chloro-3-fluoro-4-methoxyaniline and 4-chloro-3-fluoro-2-methoxy-aniline followed by demethylation of the methoxy group.

Surprisingly, when reproducing the first step of the process disclosed in the patent CN112142567, the inventors of the present invention observed only 50% conversion. As a result of the lack of reactivity of the raw material, tedious isolation and purification is required due to the formation of numerous undesired by-products.

In the light of the prior art described above, it is an object of the present invention to provide a cleaner process that does not have the aforementioned disadvantages and hence gives a selective route to 2-chloro-3-fluoro-4-alkoxy-aniline and thereby also to 2-fluoro-3-chlorophenol under mild conditions, with high yield and high purity, and where the product can be easily isolated.

The object described above was achieved by a process for preparing a compound of the general the formula (V): 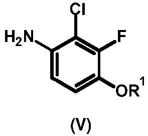
in which R¹ is C₁-C₇₋alkyl or C₇-C₁₂-arylalkyl,
characterized in that, in step (A), 1-chloro-2,3-difluorobenzene of formula (I) 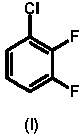
is reacted with a nitrating agent to form the compound of the formula (II) 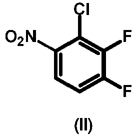 and,
that in step (B), the compound of the formula (II) is reacted with an alcohol of the general formula (III)

   R¹OH (III)
in which R¹ is as defined above,
in presence of a base and optionally a solvent
to form the compound of the general formula (IV) 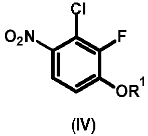
in which R¹ is as defined above; and
that in step (C), the compound of the general formula (IV) is reacted with a reducing agent A and a solvent to form the compound of the general formula (V).

Surprisingly, the inventors of the present invention have found that using 3-chloro-1,2-difluoro-4-nitrobenzene (II) instead of 1,3-dichloro-2-fluoro-4-nitrobenzene in a methoxylation reaction provides 3-chloro-2-fluoro-1-methoxy-4-nitrobenzene as a single regioisomer with 98% yield and can be used for further reactions without purification. Furthermore, 3-chloro-1,2-difluoro-4-nitrobenzene (II) can be easily obtained in one step by nitration of 1-chloro-2,3-difluorobenzene (I) in high yield and high purity. 3-chloro-1,2-difluoro-4-nitrobenzene (II) can then be further converted to the desired 2-chloro-3-fluoro-4-alkoxyaniline (V), which can be further used to make 2-fluoro-3-chlorophenol (VIII).

### General definition

Unless otherwise indicated, the expression "alkyl", in isolation or in combination with other terms, refers to linear or branched saturated hydrocarbon chains with up to 8 carbon atoms, i.e. C₁-C₈-alkyl, preferably with up to 7 carbon atoms, i.e. C₁-C₇-alkyl, very preferably with up to 4 carbon atoms, i.e. C₁-C₄-alkyl. Examples of such alkyls are methyl, ethyl, n-propyl, isopropyl, n-butyl, isobutyl, s-butyl, t-butyl, n-pentyl, n-hexyl, n-heptyl and n-octyl.

Unless otherwise indicated, the expression "arylalkyl" refers to alkyl which is substituted by aryl group. The definition C7-12-arylalkyl encompasses the widest range defined herein for an arylalkyl having a total of 7 to 12 carbon atoms in the aromatic skeleton and the alkylene chain. This definition encompasses, for example, the meanings of benzyl and phenylethyl.

### Process description

The process of the present invention is illustrated in Scheme 1 below.

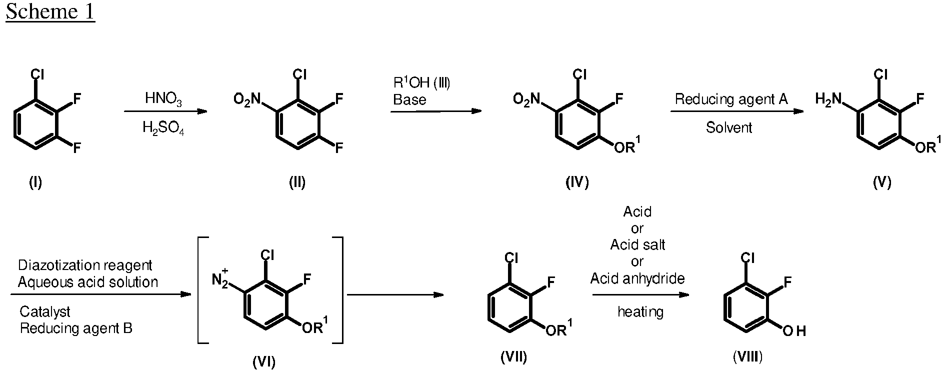

### Step A:

In step (A) of the present invention 1-chloro-2,3-difluorobenzene (I) is converted into 3-chloro-1,2-difluoro-4-nitrobenzene (II) with a nitrating agent. preferably nitrating acid.

Preferably, the nitrating acid is a combination of fuming nitric acid (HNO₃) and concentrated sulfuric acid (H₂SO₄). Preferably, the molar ratio of nitric acid to sulfuric acid is from 1:10 to 2:1.

Preferably, the molar ratio of H₂SO₄ to 1-chloro-2,3-difluorobenzene (I) is from 1:1 to 6:1, more preferably from 2:1 to 5:1, most preferably from 3:1 to 5:1.

Preferably, the molar ratio of HNO₃ to 1-chloro-2,3-difluorobenzene (I) is from 1:1 to 2:1, more preferably from 1:1 to 1.8:1, most preferably from 1.1:1 to 1.5:1.

Step (A) of the present invention can be performed at a temperature in a range between 5°C and 60 °C, preferably in a range between 20°C and 40°C.

The reaction time of the reaction in step (A) typically lies in the range from 5 hours to 12 hours.

For the isolation of the product, the reaction mixture is diluted with water and the product is extracted with organic solvent like ethyl acetate, methyl tert-butyl ether (MTBE) or CH₂Cl₂.

3-chloro-1,2-difluoro-4-nitrobenzene (II) is usually obtained in 92% purity with a yield between 85% and 92%. The latter can be used in the next step without prior purification.

The nitration can also be carried out in the presence of a solvent. Preference is given to dichloromethane or dichloroethane.

### Step B:

In step (B), 3-chloro-1,2-difluoro-4-nitrobenzene (II) is reacted with the alcohol (III) in presence of a base to produce compounds of formula (IV).

When R¹ is methyl, ethyl, n-propyl, iso-propyl, n-butyl, iso-butyl or sec-butyl, the reaction is preferably carried out in absence of a solvent other than the alcohol of formula (III) and an excess of the alcohol of the formula (III) is preferably used. When R¹ is phenethyl or benzyl, the reaction is preferably carried out in presence of a solvent.

Suitable solvents for step (B) of the present invention are, for example, aliphatic, alicyclic or aromatic hydrocarbons, for example petroleum ether, n-hexane, n-heptane, cyclohexane, methylcyclohexane, toluene, xylene or decalin, and halogenated hydrocarbons, for example, chlorobenzene, dichlorobenzene, dichloromethane, chloroform, tetrachloromethane, dichloroethane or trichloroethane, ethers such as diethyl ether, diisopropyl ether, methyl tert-butyl ether (MTBE), methyl tert-amyl ether, dioxane, Tetrahydrofuran (THF), 2-Methyltetrahydrofuran (2-Me-THF), 1,2-dimethoxyethane, 1,2-diethoxyethane or anisole; nitriles such as acetonitrile, propionitrile, n- or iso-butyronitrile or benzonitrile; amides such as dimethylformamide (DMF), dimethylacetamide (DMA), N-methylformanilide, N-Methyl-2-pyrrolidone (NMP) or hexamethylphosphoramide; sulfoxides such as dimethyl sulfoxide (DMSO) or sulfones such as sulfolane, or mixtures of the aforementioned solvents.

Preference is given to acetonitrile, toluene, and dimethylacetamide (DMA), in particular acetonitrile.

Suitable bases for step (B) of the present invention are potassium carbonate, sodium acetate, sodium alkoxides such as sodium tert-butoxide, sodium ethoxide and sodium methoxide, and potassium alkoxides such as potassium tert-butoxide, potassium ethoxide and potassium methoxide. Particularly preferred is potassium carbonate.

A particularly preferred combination of alcohol of formula (III)/base is methanol/methanoate when alcohol (III) is methanol and ethanol/ethanoate when alcohol (III) is ethanol.

The molar ratio of alcohol (III) to 3-chloro-1,2-difluoro-4-nitrobenzene (II) is preferably from 1:1 and 25:1, more preferably from 1:1 and 20:1, most preferably between 2:1 and 15:1.

Where the alcohol (III) is methanol or ethanol, a higher ratio is preferably used.

The molar ratio of base to compound (II) is preferably from 1:1 and 6:1, more preferably from 1:1 and 5:1, most preferably from 1:1 and 4:1.

Step (B) of the present invention can be performed at a temperature in a typical range between 30°C and 90 °C, preferably in a range between 40°C and 80°C, most preferably in a range between 50°C and 70°C.

The reaction time is not critical and may, according to the batch size and temperature, be selected within a range between 2h and 12h.

The product can be isolated as a solid after acidification of the reaction mixture with HCl or H₂SO₄, removal of the solvent under reduced pressure and washing with water, cold MTBE (methyl tert-butyl ether) or isopropanol.

Typical yields for step (B) of the present invention are in the range of 82% to 95 %.

### Step C:

In step (C), compounds of formula (IV) are converted into compounds of formula (V) in presence of a reducing agent A and a solvent.

Suitable reducing agents A include SnCl₂/HCl, Fe/HCl, Fe/NH₄Cl, Zn/HCl, Zn/NH₄Cl, H₂/Pd-C, H₂/Pt-C and H₂/Raney Ni. Preferably, the reducing agent A is hydrogen in combination with a hydrogenation catalyst selected from the group consisting of palladium on carbon, Raney Nickel and platinum on carbon.

Suitable solvents for step (C) of the present invention are acetic acid and alcohols including but not limited to methanol, ethanol, isopropanol, n-propanol and n-butanol. Preference is given to acetic acid, methanol, ethanol and isopropanol.

Preferably when the reducing agent A is hydrogen, a catalyst is added in an amount of 0,1-10 mol % and more preferably in an amount of 0,1-5 mol %, in each case based on the molar amount of the compound of formula (IV). Suitable catalysts for hydrogenation reduction are palladium on carbon, Raney Nickel and, platinum on carbon. Most preferred are platinum on carbon 1 % and Raney Nickel.

Step (C) of the present invention proceeds usually between 1 and 20 bars, preferably between 1 and 10 bars. Preferably when the reducing agent is hydrogen, the reaction is carried out under a hydrogen pressure of between 2 and 5 bar.

Step (C) of the present invention can be performed at a temperature in a typical range between 10°C and 40 °C, preferably at room temperature.

The reaction time typically lies in the range from 1 hour to 10 hours.

Typically, the yield for step (C) of the present invention is more than 90%.

The present invention also relates to a process for preparing 2-fluoro-3-chlorophenol (VIII) 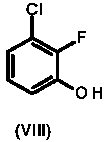 comprising the process for preparing the compound of the formula (V) according to the invention, and further comprising step (D) reacting the compound of the formula (V) obtained in step (C) with a diazotization reagent in presence of an aqueous acid solution together with a catalyst and a reducing agent B to form a compound of the general formula (VII) 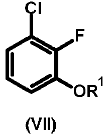
in which R¹ is C₁-C₇-alkyl or C₇-C₁₂-arylalkyl;
and the latter is converted, in step (E), into 2-fluoro-3-chlorophenol (VIII) upon heating in presence of an acid, acid salt or acid anhydride.

### Step D:

In step (D), compounds of formula (V) are converted into compounds of formula (VII) in presence of a diazotization reagent, an aqueous acid solution, a catalyst and a reducing agent B *via* the *in-situ* formation of diazonium salt of formula (VI).

Suitable diazotization reagents are sodium nitrite, potassium nitrite, nitrous acid, nitrosyl sulfuric acid, methyl nitrite and n-butyl nitrite. Most preferred diazotization reagents are sodium nitrite and potassium nitrite.

Suitable acids for the formation of the diazonium salts (VI) are selected from the group consisting of hydrochloric acid, sulfuric acid, nitric acid, phosphoric acid, acetic acid, trifluoroacetic acid, hydrofluoric acid, and hydrobromic acid. Preference is given to hydrochloric acid (HCl) and sulfuric acid (H₂SO₄).

Suitable catalysts are copper (I) chloride, copper(I) bromide, copper (II) sulfate, cuprous oxide (copper(I) oxide), copper oxide (copper (II) oxide), copper nitrate, potassium trifluoroacetate, sodium trifluoroacetate, trifluoroacetic acid, copper acetate, copper carbonate, sodium acetate, and potassium acetate. Most preferred catalysts are copper(I) chloride and copper(I) bromide.

Preferred reducing agents B include sodium hypophosphite, potassium hypophosphite, hypophosphorous acid, ethanol, methanol and isopropanol. Most preferred reducing agents are isopropanol and sodium hypophosphite.

The molar ratio of diazotization reagent to compound of formula (V) is preferably from 1:1 to 2:1, more preferably from 1.1:1 to 1.5:1.

The molar ratio of acid to compound of formula (V) is preferably from 2:1 to 20:1, more preferably from 2:1 to 10:1.

The molar ratio of catalyst to compound of formula (V) is preferably from 0.01:1 to 0.1:1.

The molar ratio of reduction agent B to compound of formula (V) is preferably from 1:1 to 15:1, more preferably from 2:1 to 10:1.

Step (D) of the present invention can be performed at a temperature in a range between 0°C and 20 °C, preferably in a range between 0°C and 10°C, most preferably in a range between 0°C and 5°C.

The reaction time typically lies in the range from 2 hours to 10 hours.

Typically, the yield for step (D) of the present invention is in the range of 78-90%.

### Step E:

In step (E), compounds of formula (VII) are converted into 2-fluoro-3-chlorophenol (VIII) upon heating in presence of an acid, an acid salt or an acid anhydride.

Preferred acids are hydrochloric acid, hydrobromic acid, hydroiodic acid, hydrofluoric acid, acetic acid, sulfuric acid and boron tribromide.

Preferred acid salts are pyridine hydrobromide and pyridine hydrochloride.

A preferred acid anhydride is acetic anhydride.

Particularly preferred are hydrobromic acid as 50 % water solution, HCl as 37 % water solution, pyridine hydrochloride and boron tribromide.

The molar ratio of acid or its salt to compound of formula (VII) is preferably from 1:1 to 30:1, more preferably from 1:1 to 25:1, most preferably from 1:1 to 20:1.

The reaction temperature of step (E) of the present invention is typically within a temperature range from 50 °C to 150°C, preferably at temperatures of 70 °C to 130 °C, more preferably at 90 °C to 120 °C.

Step (E) of the present invention is generally performed under standard pressure. Alternatively, however, it is also possible to work under pressure in an autoclave.

The compound 2-fluoro-3-chlorophenol (VIII) from step (E) of the present invention can be extracted directly from the reaction mixture with organic solvent.

The reaction time typically lies in the range from 3 hours to 8 hours.

Typically, the yield for step (E) of the present invention is in the range of 70-90%.

More preferred is a process according to the present invention, where the radical R¹ in formula (IV) to (VII) is defined as follows:
R¹ is methyl, ethyl, *n*-propyl, *iso*-propyl, *n*-butyl, *iso*-butyl, *sec-*butyl*,* phenethyl or benzyl.

Most preferred is a process according to the present invention, where the radical R¹ in formula (IV) to (VII) is defined as follows:
R1 is methyl, ethyl, *iso*-propyl or benzyl.

The invention is illustrated by the following examples:

### EXAMPLES

### Example 1

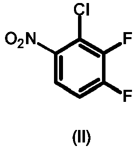

### 3-Chloro-1,2-difluoro-4-nitrobenzene (II)

To the mixture of 19,4 g sulfuric acid (96 % purity, d 1,96) and 4 g of nitric acid (d 1,51, purity 100 %), 9,5 g of 1-chloro-2,3-difluorobenzene (I) was added within 1 h at 20-25°C. The mixture was stirred for 5 h at 30°C and poured on ice. The product was extracted with 200 ml of CH₂Cl₂ washed with water and the solvent was evaporated to give 11,6 g of pale-yellow oil with purity w.w. 92%. Yield = 85%, adjusted to the purity.

**¹H NMR** (400 MHz, DMSO-d₆) δ 7,7 (ddd, 1H); 8,1 (ddd, 1H) ppm.

**Mass spectra (ESI⁺, m/z)** [M+H] 194.

### Example 2

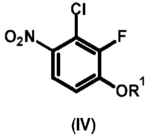

### Example 2a: 3-Chloro-2-fluoro-1-methoxy-4-nitrobenzene (IV-a with R¹ = Methyl)

A solution of 19,3 g of 3-Chloro-1,2-difluoro-4-nitrobenzene (II) and 10,8 g of sodium methoxide in 150 ml methanol were heated for 2 h at 65°C. The mixture was cooled and acidified with 20 ml of 20% hydrochloric acid. The solvent and volatile products were removed in vacuum, 100 ml of cold methyl tert-butyl ether were added and the formed solid was filtered off, washed with water and cold MTBE to yield 20 g (98%) of white solid with m.p. of 74-75°C.

**¹H NMR** (400 MHz, DMSO-d₆) δ 4,0 (s, 3H); 7,4 (d, 1H); 8,1 (d, 1H) ppm.

**Mass spectra (ESI⁺, m/z)** [M+H] 206.

### Example 2b: 3-Chloro-2-fluoro-1-methoxy-4-nitrobenzene (IV-a with R¹ = Methyl)

### Comparative Example (CN 112142567):

21 g of 1,3-dichloro-2-fluoro-4-nitrobenzene (prepared according to CN 112047804, were placed in 50 ml methanol and heated to 40°C. 28,3 g of 20% solution of sodium methoxide were added dropwise and the mixture was stirred for 2 h at 50°C. The mixture was cooled to 20 °C and pH was adjusted to 7 upon addition of hydrochloric acid. The mixture was filtered to remove the salt and the solvent was evaporated in vacuum. The obtained oil (21 g) was analyzed using LC/MS showing a mixture of 6-7 components with 50 area % of the starting materials. The isolation of the pure product was not possible.

### Example 2c: 3-chloro-2-fluoro-1-benzyloxy-4-nitrobenzene (IV-b with R¹ = benzyl)

A solution of 19,3 g of 3-Chloro-1,2-difluoro-4-nitrobenzene, 21,6 g of benzyl alcohol and 27,6 of potassium carbonate in 100 ml acetonitrile were heated for 12 h at 65°C. The mixture was cooled and acidified with 50 ml of 10 % hydrochloric acid. The solvent and volatile products were removed in vacuum, and 100 ml of cold isopropanol were added. The formed solid was filtered off, washed with water and cold isopropanol to give 23 g (82 %) of white solid with m.p. of 83°C.

**¹H NMR** (400 MHz, DMSO-d₆) δ 5,4 (s, 2H); 7,3-7,5 (m, 6H); 8,1 (d, 1H) ppm

**Mass spectra (ESI⁺, m/z)** [M+H] 282.

### Example 3

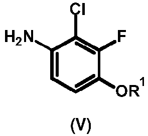

A solution of 100 mmol of compound (IV) in 100 ml of methanol and 0,4 g of catalyst (1% platinum on activated carbon) were placed in an autoclave. The autoclave was purged with hydrogen and then a hydrogen pressure of 5 bar was applied. The mixture was stirred in the autoclave at room temperature for 5 h. Then, the reaction mixture was poured through filter paper to remove the catalyst. The solvent was removed in vacuum.

### Example 3a: 2-chloro-3-fluoro-4-methoxyaniline (V-a with R¹ = Methyl)

Compound V-a was obtained as a white solid (16,6 g; 95 % yield).

**¹H NMR** (400 MHz, DMSO-d₆) δ 3,7 (s, 3H); 5,3 (b.s., 2H); 6,55 (m, 1H); 6,8 (m, 1H) ppm.

**Mass spectra (ESI⁺, m/z)** [M+H] 176.

### Example 3b: 2-chloro-3-fluoro-4-benzyloxy-aniline (V-b with R¹ = Benzyl)

Compound V-b was obtained as an oil (95% yield).

**¹H NMR** (400 MHz, DMSO-d₆) δ 5,1 (b.s, 2H); 5,35 (s, 2H); 7,1-7,6 (m, 7H) ppm.

**Mass spectra (ESI⁺, m/z)** [M+H] 252.

### Example 4

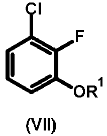

A mixture of 100 mmol (V) and 50 ml of 10% hydrochloric acid were stirred for 30 min at 20°C to form a homogeneous slurry followed by addition of 0,4 g CuCl in 50 ml HCl and 60 ml of isopropanol. The mixture was stirred for 30 min and cooled to 0°C. An aqueous solution of sodium nitrite (14 ml, 40 %) was slowly added at 0°C to this mixture. The temperature was adjusted to 20°C and the reaction mixture stirred for 10 h. 100 ml of water were added to the mixture and the oily product was extracted with methyl tert-butyl ether. The organic phase was washed with water, dried and evaporated to give the desired compound of formula (VII).

### Example 4a: 1-chloro-2-fluoro-3-methoxybenzene (VII-a with R¹ = Methyl).

Compound VII-a was obtained as a pale-yellow oil (13,6 g; 85% yield).

**¹H NMR** (400 MHz, DMSO-d₆) δ 3,8 (s, 3H); 7,0-7,2 (m, 3H) ppm.

**¹⁹F NMR** (376,4 MHz, DMSO-d₆) δ -137,9 ppm.

**Mass spectra (ESI⁺, m/z)** [M+H] 161.

### Example 4b: 1-benzyloxy-3-chloro-2-fluorobenzene (VII-b with R¹ = Benzyl).

Compound VII-b was obtained as an oil in 78 % yield.

**¹H NMR** (400 MHz, DMSO-d₆) δ 5,3 (s, 2H); 7,2-7,5 (m, 8H) ppm.

**¹⁹F NMR** (376,4 MHz, DMSO-d₆) δ -137,7 ppm.

**Mass spectra (ESI⁺, m/z)** [M+H] 237.

### Example 5

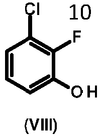

### 2-fluoro-3-chloro-phenol (VIII)

A mixture of 50 g of 1-chloro-2-fluoro-3-methoxybenzene and 100 ml of HCl (37 % water solution) were heated in a Parr Glass Autoclave at 110°C. After 7 h, the mixture was cooled, and the product extracted 3 times with 100 ml CH₂Cl₂. The solvent was evaporated to give 40 g of 2-fluoro-3-chloro-phenol as a pale-yellow liquid.

**¹H NMR** (400 MHz, DMSO-d₆) δ 6,91-6,95 (m, 2H); 7,00 (s, 1H); 10,3 (s, 1H) ppm.

**¹⁹F NMR** (376,4 MHz, DMSO-d₆) δ -139,9 ppm.

**Mass spectra (ESI⁺, m/z)** [M+H] 145.

## Claims

1. A process for preparing a compound of the general formula (V): 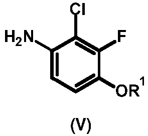
in which R¹ is C₁-C₇₋alkyl or C₇-C₁₂-arylalkyl,
**characterized in that**, in step (A), 1-chloro-2,3-difluorobenzene of formula (I) 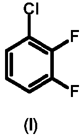
is reacted with a nitrating agent to form the compound of the formula (II) 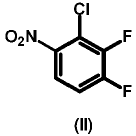 and,
that in step (B), the compound of the formula (II) is reacted with an alcohol of the general formula (III)
R¹OH (III)
in which R¹ is as defined above,
in presence of a base and optionally a solvent
to form the compound of the general formula (IV) 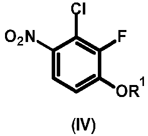
in which R¹ is as defined above; and
that in step (C), the compound of the general formula (IV) is reacted with a reducing agent A and a solvent to form the compound of the general formula (V).

2. The process according to claim 1, wherein R¹ is methyl, ethyl, *n*-propyl, *iso*-propyl, *n-*butyl*, iso-*butyl, *sec*-butyl, phenethyl or benzyl.

3. The process according to claim 1 or 2, wherein in step (A), the nitrating agent is a combination of fuming nitric acid and concentrated sulfuric acid in a molar ratio of nitric acid to sulfuric acid from 1:10 to 2:1.

4. The process according to any of claims 1 to 3, wherein in step (B), the base is selected from group consisting of potassium carbonate, sodium acetate, sodium alkoxides such as sodium *tert-*butoxide, sodium ethoxide and sodium methoxide, and potassium alkoxides such as potassium *tert*-butoxide, potassium ethoxide and potassium methoxide.

5. The process according to any of claims 1 to 4, wherein in step (B), the molar ratio of base to compound (II) is from 1:1 to 6:1.

6. The process according to any of claims 1 to 5, wherein in step (C), the reducing agent A is hydrogen in combination with a hydrogenation catalyst selected from the group consisting of palladium on carbon, Raney Nickel and platinum on carbon.

7. The process according to any of claim 1 to 6, wherein in step (C), the hydrogenation catalyst is added in an amount of 0,1-10 mol%.

8. The process according to any of claims 1 to 7, wherein in step (C), the solvent used is selected from acetic acid, methanol, ethanol, and isopropanol.

9. A process for preparing 2-fluoro-3-chlorophenol (VIII) 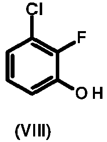
comprising the process for preparing the compound of the formula (V) according to any of claims 1 to 8, and further comprising step (D) reacting the compound of the formula (V) obtained in step (C) with a diazotization reagent in presence of an aqueous acid solution together with a catalyst and a reducing agent B to form a compound of the general formula (VII) 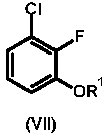
in which R¹ is C₁-C₇-alkyl or C₇-C₁₂-arylalkyl;
and the latter is converted, in step (E), into 2-fluoro-3-chlorophenol (VIII) upon heating in presence of an acid, acid salt or acid anhydride.

10. The process according to claim 9, wherein R¹ is methyl, ethyl, *n*-propyl, *iso*-propyl, *n-*butyl*, iso-*butyl, *sec*-butyl, phenethyl or benzyl.

11. The process according to claim 9 or 10, wherein in step (D), the catalyst is copper(I) chloride or copper(I) bromide.

12. The process according to any of claims 9 to 11, wherein in step (D), the reducing agent B is selected in the group consisting of sodium hypophosphite, potassium hypophosphite, hypophosphorous acid, ethanol, methanol and isopropanol.

13. The process according to any of claims 9 to 12, wherein in step (D), the diazotization compound is selected from sodium nitrite, potassium nitrite, nitrous acid, nitrosyl sulfuric acid, methyl nitrite and n-butyl nitrite.

14. The process according to any of claims 9 to 13, wherein in step (D), the acid is selected from the group consisting of hydrochloric acid, sulfuric acid, nitric acid, phosphoric acid, acetic acid, trifluoroacetic acid, hydrofluoric acid, and hydrobromic acid.

15. The process according to any of claims 9 to 14, wherein in step (E) the acid is selected from hydrochloric acid, hydrobromic acid, hydroiodic acid, hydrofluoric acid, acetic acid, sulfuric acid and boron tribromide; the acid salt is selected from pyridine hydrobromide and pyridine hydrochloride; and the acid anhydride is acetic anhydride.

## Patentansprüche

1. Ein Verfahren zur Herstellung einer Verbindung der allgemeinen Formel (V): 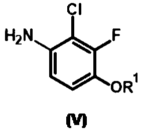
wobei R¹ C₁-C₇₋alkyl oder C₇-C₁₂-Arylalkyl ist,
**dadurch gekennzeichnet, dass** in Schritt (A) 1-Chlor-2,3-difluorbenzol der Formel (I) 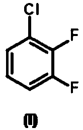
mit einem Nitrierungsmittel umgesetzt wird, um die Verbindung der Formel (II) zu bilden 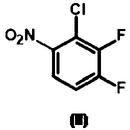 und,
dass in Schritt (B) die Verbindung der Formel (II) mit einem Alkohol der allgemeinen Formel (III)
**R¹OH** **(■)**
wobei R¹ wie oben definiert ist,
im Vorliegen einer Base und optional eines Lösungsmittels
umgesetzt wird, um die Verbindung der allgemeinen Formel (IV) zu bilden 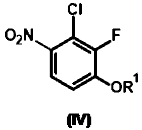
wobei R¹ wie oben definiert ist; und
dass in Schritt (C) die Verbindung der allgemeinen Formel (IV) mit einem Reduktionsmittel A und einem Lösungsmittel umgesetzt wird, um die Verbindung der allgemeinen Formel (V) zu bilden.

2. Das Verfahren nach Anspruch 1, wobei R¹ Methyl, Ethyl, *n*-Propyl, *iso*-Propyl, *n*-Butyl, *iso*-Butyl, *sec*-Butyl, Phenethyl oder Benzyl ist.

3. Das Verfahren nach Anspruch 1 oder 2, wobei in Schritt (A) das Nitrierungsmittel eine Kombination aus rauchender Salpetersäure und konzentrierter Schwefelsäure in einem molaren Verhältnis von Salpetersäure zu Schwefelsäure von 1:10 bis 2:1 ist.

4. Das Verfahren nach einem der Ansprüche 1 bis 3, wobei in Schritt (B) die Base ausgewählt ist aus der Gruppe bestehend aus Kaliumcarbonat, Natriumacetat, Natriumalkoxiden wie Natrium-tert-butoxid, Natriumethoxid und Natriummethoxid sowie Kaliumalkoxiden wie Kalium-tert-butoxid, Kaliumethoxid und Kaliummethoxid.

5. Das Verfahren nach einem der Ansprüche 1 bis 4, wobei in Schritt (B) das molare Verhältnis von Base zu Verbindung (II) 1:1 bis 6:1 beträgt.

6. Das Verfahren nach einem der Ansprüche 1 bis 5, wobei in Schritt (C) das Reduktionsmittel A Wasserstoff in Kombination mit einem Hydrierkatalysator ist, der aus der Gruppe bestehend aus Palladium auf Kohlenstoff, Raney-Nickel und Platin auf Kohlenstoff ausgewählt ist.

7. Das Verfahren nach einem der Ansprüche 1 bis 6, wobei in Schritt (C) der Hydrierkatalysator in einer Menge von 0,1-10 mol% zugegeben wird.

8. Das Verfahren nach einem der Ansprüche 1 bis 7, wobei in Schritt (C) das verwendete Lösungsmittel aus Essigsäure, Methanol, Ethanol und Isopropanol ausgewählt ist.

9. Ein Verfahren zur Herstellung von 2-Fluor-3-chlorphenol (VIII) 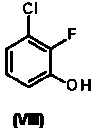
umfassend das Verfahren zur Herstellung der Verbindung der Formel (V) nach einem der Ansprüche 1 bis 8 und ferner umfassend Schritt (D), bei dem die in Schritt (C) erhaltene Verbindung der Formel (V) mit einem Diazotierungsreagenz im Vorliegen einer wässrigen Säurelösung zusammen mit einem Katalysator und einem Reduktionsmittel B umgesetzt wird, um eine Verbindung der allgemeinen Formel (VII) zu bilden 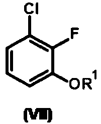
wobei R¹ C₁-C₇-alkyl oder C₇-C₁₂-Arylalkyl ist;
und wobei letztere in Schritt (E) durch Erhitzen in Gegenwart einer Säure, eines Säuresalzes oder eines Säureanhydrids in 2-Fluor-3-chlorphenol (VIII) überführt wird.

10. Das Verfahren nach Anspruch 9, wobei R¹ Methyl, Ethyl, *n*-Propyl, *iso*-Propyl, *n*-Butyl, *iso*-Butyl, *sec*-Butyl, Phenethyl oder Benzyl ist.

11. Das Verfahren nach Anspruch 9 oder 10, wobei in Schritt (D) der Katalysator Kupfer(I)-chlorid oder Kupfer(I)-bromid ist.

12. Das Verfahren nach einem der Ansprüche 9 bis 11, wobei in Schritt (D) das Reduktionsmittel B aus der Gruppe bestehend aus Natriumhypophosphit, Kaliumhypophosphit, hypophosphoriger Säure, Ethanol, Methanol und Isopropanol ausgewählt ist.

13. Das Verfahren nach einem der Ansprüche 9 bis 12, wobei in Schritt (D) die Diazotierungsverbindung aus Natriumnitrit, Kaliumnitrit, salpetriger Säure, Nitrosylschwefelsäure, Methylnitrit und n-Butylnitrit ausgewählt ist.

14. Das Verfahren nach einem der Ansprüche 9 bis 13, wobei in Schritt (D) die Säure aus der Gruppe bestehend aus Salzsäure, Schwefelsäure, Salpetersäure, Phosphorsäure, Essigsäure, Trifluoressigsäure, Fluorwasserstoffsäure und Bromwasserstoffsäure ausgewählt ist.

15. Das Verfahren nach einem der Ansprüche 9 bis 14, wobei in Schritt (E) die Säure aus Salzsäure, Bromwasserstoffsäure, lodwasserstoffsäure, Fluorwasserstoffsäure, Essigsäure, Schwefelsäure und Bortribromid ausgewählt ist; das Säuresalz aus Pyridinhydrobromid und Pyridinhydrochlorid ausgewählt ist; und das Säureanhydrid Essigsäureanhydrid ist.

## Revendications

1. Procédé de préparation d'un composé de la formule générale (V) : 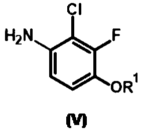
dans lequel R¹ est un alkyle en C₁-C₇ ou un arylalkyle en C₇-C₁₂,
**caractérisé en ce que**, à l'étape (A), le 1-chloro-2,3-difluorobenzène de formule (I) 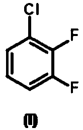
réagit avec un agent de nitration pour former le composé de formule (II) 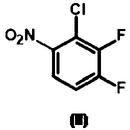 et,
qu'à l'étape (B), le composé de formule (II) réagit avec un alcool de formule générale (III)
**R¹OH** **(■)**
dans lequel R¹ est tel que défini ci-dessus,
en présence d'une base et éventuellement d'un solvant
pour former le composé de formule générale (IV) 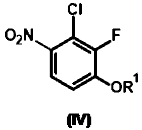
dans lequel R¹ est tel que défini ci-dessus ; et
qu'à l'étape (C), le composé de formule générale (IV) réagit avec un agent réducteur A et un solvant pour former le composé de formule générale (V).

2. Le procédé selon la revendication 1, dans lequel R¹ est méthyle, éthyle, *n*-propyle, *iso*-propyle, *n*-butyle, *iso*-butyle, *sec*-butyle, phénéthyle ou benzyle.

3. Le procédé selon la revendication 1 ou 2, dans lequel à l'étape (A), l'agent de nitration est une combinaison d'acide nitrique fumant et d'acide sulfurique concentré dans un rapport molaire acide nitrique/acide sulfurique allant de 1:10 à 2:1.

4. Le procédé selon l'une quelconque des revendications 1 à 3, dans lequel à l'étape (B), la base est sélectionnée parmi le group constitant en carbonate de potassium, acétate de sodium, alcoolates de sodium tels que le tert-butoxyde de sodium, l'éthoxyde de sodium et le méthoxyde de sodium, et alcoolates de potassium tels que le tert-butoxyde de potassium, l'éthoxyde de potassium et le méthoxyde de potassium.

5. Le procédé selon l'une quelconque des revendications 1 à 4, dans lequel à l'étape (B), le rapport molaire de la base au composé (II) va de 1:1 à 6:1.

6. Le procédé selon l'une quelconque des revendications 1 à 5, dans lequel à l'étape (C), l'agent réducteur A est l'hydrogène en combinaison avec un catalyseur d'hydrogénation sélectionné parmi le group constitant en palladium sur charbon, nickel de Raney et platine sur charbon.

7. Le procédé selon l'une quelconque de la revendication 1 à 6, dans lequel à l'étape (C), le catalyseur d'hydrogénation est ajouté en une quantité de 0,1 à 10 mol%.

8. Le procédé selon l'une quelconque des revendications 1 à 7, dans lequel à l'étape (C), le solvant utilisé est sélectionné parmi l'acide acétique, le méthanol, l'éthanol et l'isopropanol.

9. Procédé de préparation du 2-fluoro-3-chlorophénol (VIII) 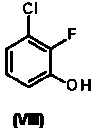
comprenant le procédé de préparation du composé de formule (V) selon l'une quelconque des revendications 1 à 8, et comprenant en outre l'étape (D) consistant à faire réagir le composé de formule (V) obtenu à l'étape (C) avec un réactif de diazotation en présence d'une solution acide aqueuse avec un catalyseur et un agent réducteur B pour former un composé de formule générale (VII) 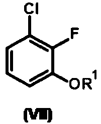
dans lequel R¹ est un alkyle en C₁-C₇ ou un arylalkyle en C₇-C₁₂ ;
et ce dernier est converti, à l'étape (E), en 2-fluoro-3-chlorophénol (VIII) par chauffage en présence d'un acide, d'un sel acide ou d'un anhydride d'acide.

10. Le procédé selon la revendication 9, dans lequel R¹ est méthyle, éthyle, *n*-propyle, *iso*-propyle, *n*-butyle, *iso*-butyle, *sec*-butyle, phénéthyle ou benzyle.

11. Le procédé selon la revendication 9 ou 10, dans lequel à l'étape (D), le catalyseur est le chlorure de cuivre(I) ou le bromure de cuivre(I).

12. Le procédé selon l'une quelconque des revendications 9 à 11, dans lequel à l'étape (D), l'agent réducteur B est sélectionné dans le groupe constituée de hypophosphite de sodium, hypophosphite de potassium, acide hypophosphoreux, éthanol, méthanol et isopropanol.

13. Le procédé selon l'une quelconque des revendications 9 à 12, dans lequel à l'étape (D), le composé de diazotation est sélectionné parmi le nitrite de sodium, le nitrite de potassium, l'acide nitreux, l'acide nitrosylsulfurique, le nitrite de méthyle et le nitrite de n-butyle.

14. Le procédé selon l'une quelconque des revendications 9 à 13, dans lequel à l'étape (D), l'acide est sélectionné parmi le group constitant en acide chlorhydrique, acide sulfurique, acide nitrique, acide phosphorique, acide acétique, acide trifluoroacétique, acide fluorhydrique et acide bromhydrique.

15. Le procédé selon l'une quelconque des revendications 9 à 14, dans lequel à l'étape (E) l'acide est sélectionné parmi l'acide chlorhydrique, l'acide bromhydrique, l'acide iodhydrique, l'acide fluorhydrique, l'acide acétique, l'acide sulfurique et le tribromure de bore ; le sel acide est sélectionné parmi le bromhydrate de pyridine et le chlorhydrate de pyridine ; et l'anhydride d'acide est l'anhydride acétique.
